(19) **Europäisches Patentamt**
**European Patent Office**
**Office européen des brevets**

(11) Veröffentlichungsnummer: **0 085 923**
**B2**

(12) **NEUE EUROPÄISCHE PATENTSCHRIFT**

(45) Veröffentlichungstag der neuen Patentschrift:
**23.01.91**

(51) Int. Cl.⁵: **A 61 L 15/16, A 61 K 35/16**

(21) Anmeldenummer: **83100869.3**

(22) Anmeldetag: **31.01.83**

(54) **Fibrinogenzubereitung, Verfahren zu ihrer Herstellung und ihre Verwendung.**

(30) Priorität: **04.02.82 DE 3203775**

(43) Veröffentlichungstag der Anmeldung:
**17.08.83 Patentblatt 83/33**

(45) Bekanntmachung des Hinweises auf die
Patenterteilung:
**15.10.86 Patenblatt 86/42**

(45) Bekanntmachung des Hinweises auf die
Entscheidung u̇ber den Einspruch:
**23.01.91 Patentblatt 91/04**

(84) Benannte Vertragsstaaten:
**AT BE CH DE FR GB IT LI LU NL SE**

(56) Entgegenhaltungen:
EP-B-0 035 616     DE-A-3 002 934
DE-A-3 001 435     DE-A-24 619 69
DE-A-3 002 933

E. Michalyi "Properties of Fibrin dissolved in
Urea solutions" Acta chem. Scand. 4 344-
250(1950)

Die Akte enthält technische Angaben, die nach
dem Eingang der Anmeldung eingereicht
wurden und die nicht in dieser Patentschrift
enthalten sind.

(73) Patentinhaber: **BEHRINGWERKE**
**Aktiengesellschaft**
**Postfach 1140**
**D-3550 Marburg 1 (DE)**

(72) Erfinder: **Fuhge, Peter, Dr.**
**Mühlenstrasse 10**
**D-3551 Lahntal (DE)**
Erfinder: **Heimburger, Norbert, Prof. Dr.**
**Sonnenhang 10**
**D-3550 Marburg 1 (DE)**
Erfinder: **Stöhr, Hans-Arnold**
**Schulstrasse 66**
**D-3552 Wetter (DE)**
Erfinder: **Burk, Wolfgang**
**Mittelstrasse 2**
**D-3554 Gladenbach-Mornshausen (DE)**

(74) Vertreter: **Klein, Otto, Dr. et al**
**Hoechst AG Zentrale Patentabteilung Postfach
80 03 20**
**D-6230 Frankfurt am Main 80 (DE)**

(56) References cited:
**Monmaerts "On the nature of forces operating
in blood clotting II. The clotting of fibrinogen as
a two-step reaction" J. Gen. Physiol. 29 113-122
(1946)**

Courier Press, Leamington Spa, England.

**EP 0 085 923 B2**

(56) References cited:

Husain et al. "Rapid purification of a high-affinity plasminogen activator from human blood plasma by specific adsorption on fibrin/Celite" Proc. Nat. Acad. Science USA 78 4265-69 (1981)

Vuento et al. "Dissociation of fibronectin from gelatine agarose by amino compounds"

Biochem J 175, 333-336 (1978)

Rote Liste 1980 47020 Ca

## Beschreibung

Die Erfindung betrifft eine feste Fibrinogenzubereitung und ein Verfahren zu ihrer Herstellung. Diese Zubereitung kann zur Herstellung einer Fibrinogenlösung verwendet werden, die als Kleber für menschliche und tierische Gewebe geeignet ist.

Es ist bekannt, daß lyophilisiertes Fibrinogen und Lyophilisate von Plasmaproteinben, die einen hohen Anteil Fibrinogen enthalten, nur langsam, bei erhöhter Temperatur und nur in geringer Konzentration wieder in Lösung zu bringen sind.

Seit einiger Zeit wird Fibrinogen als physiologische Klebesubstanz bei der Versorgung von Verletzungen parenchymatöser Organe, Knochen oder Gefäße verwendet. Für diese Indikation ist est notwendig, das Fibrinogen in möglichst hoher Konzentration zu lösen, weil die Klebewirkung eine Funktion der Fibrinogenkonzentration ist. Da Fibrinogenlösungen mit dem erforderlichen Gehalt von 8 bis 10% gerinnbarem Fibrinogen aus Fibrinogenlyophilisaten nur schwierig herstellbar sind, verwendet man tiefgefrorenes fibrinogenhaltiges sogenanntes Kryopräzipität für die Gewebeklebung.

Diese Verwendung von tiefgefrorenem Kryoprazipitat ist jedoch mit Nachteilen behaftet, da solche Präparate instabil sind und bis zum Gebrauch auf einer Temperatur unterhalb −20°C gehalten werden müssen.

Eine feste Fibrinogenzubereitung, die sich zu einer Fibrinogenlösung mit mindestens 7 g/100 ml auflösen läßt, wäre daher von großem Vorteil.

Weiter kann solch eine Fibrinogenlösung in einer niedrigeren Konzentration von ca. 2% Fibrinogen als intravenöses Präparat für Situationen des akuten Verbrauchs von Fibrinogen bei Patienten mit verschiedenen Erkrankungen eingesetzt werden. Hierzu werden heute Fibrinogenkonzentrate eingesetzt, die aus Kryopräzipitat hergestellt werden und verschiedene Plasmaproteine zusätzlich enthalten. Reines Fibrinogen wird dagegen heutzutage nicht eingesetzt.

Aufgabe der vorliegenden Erfindung ist es daher, eine Zubereitung auf der Basis von reinem Fibrinogen zur Verfügung zu stellen, die sich aufgrund ihrer Eigenschaften sowohl als Fibrinkleber als auch als Fibrinogenkonzentrat für intravenöse Applikation beim Menschen einsetzen läßt und eine gute Löslichkeit aufweist.

Ein Fibrinogenlyophilisat, das als Gewebeklebstoff geeignet sein soll, ist aus der deutschen Offenlegungsschrift 30 02 934 bekannt. Bei diesem Lyophilisat handelt es sich ebenfalls um ein Kryopräzipitat, das dementsprechend außer Fibrinogen und Faktor XIII auch noch Plasminogen, Albumin und andere Plasmabestandteile enthält. Zur Stabilisierung des Lyophilisates und der rekonstituierten Lösung wird ein Plasminogen-Aktivator-Inhibitor zugefügt. Dieses Lyophilisat ist schlecht und nur bei erhöhter Temperatur (37°C) löslich. Nach Rekonstitution ist das Produkt max. 4 Stunden bei Raumtemperatur stabil.

Es wurde nun eine lyophilisierte Fibrinogen-Zubereitung gefunden, die zur Stabilisierung weder einen Plasminogen-Aktivator-Inhibitor noch Albumin zu enthalten braucht, und die zur Herstellung hochkonzentrierter Fibrinogenlösungen (etwa 8%) auch bei Raumtemperatur geeignet ist. Es ist nicht nötig, als Ausgangspunkt Kryopräzipitat einzusetzen, da auch der Einsatz von Reinifibrinogen als Ausgangsprodukt möglich ist.

Dies wurde durch den überraschenden Befund möglich, daß durch Zusatz von Arginin die Löslichkeit von Fibrinogenlyophilisaten erhöht und die für die Verarbeitbarkeit bedeutsame Viskosität daraus hergestellter Lösungen gesenkt wird.

Diese Effekte können dadurch noch verbessert werden, daß man die im Ausgangsprodukt enthaltenen Fibrinogenpolymere abscheidet, so daß nicht nur Kryopräzipitate als Ausgangsmaterial zu verwenden sind, sondern auch andere Fibrinogenniederschläge. Damit ist beispielsweise eine ökonomischere Ausnutzung des teuren Kryopräzipitates möglich: So können aus einem Kryopräzipitat neben F VIII-Konzentrat auch Fibrinogenkonzentrate hergestellt werden.

Gegenstand der Erfindung ist demnach eine feste Fibrinogen-Zubereitung, die Arginin enthält.

Es wird bevorzugt in einer solchen Menge zugesetzt, daß sein Gehalt in der Zubereitung 0.05 bis 5 Gewichtsprozent ist.

Die Zubereitung wird getrocknet, vorzugsweise lyophilisiert.

Zur weiteren Verbesserung der Löslichkeit einer solchem Fibrinogen-Zubereitung kann ihr eine Aminosäure mit hydrophober Seitenkette z.B. L-Leucin oder eine wasserlösliche Fettsäure z.B. Buttersäure in einer Menge von je 0,1 bis 5 Gewichtsprozent zugesetzt werden.

Eine Verbesserung der Lösungsgeschwindigkeit kann dadurch erreicht werden, daß der Gasraum über der festen Fibrinogen-Zubereitung mit mindestens 20 Volumprozent Kohlendioxid gefüllt wird. Der Rest der Gesatmosphäre kann Stickstoff, ein anderes Inertgas oder Luft sein.

Die Zubereitung kann weiterhin noch Albumin enthalten.

Bei Verwendung als Fibrinkleber kann außerdem zur Erhöhung der Reißfestigkeit im beschriebenen Rattenhautreißtest Faktor XIII aus menschlichem Plasma oder Placenta hinzugefügt werden. Eine Konzentration zwischen 40—60 E Faktor XIII/ml Fibrinogenkonzentrat erwies sich als optimal.

Der Zusatz eines Fibrinolyseinhibitors, z.B. Aprotinin, kann bei Auflösung des Fibrinogenkonzentrats erfolgen: Ein Fibrinogenkonzentrat mit 60 E Faktor XIII, 1% Albumin und 8% gerinnbarem Fibrinogen wird z.B. mit einer Aprotininlösung mit 1 000 Kallikrein-Inhibitor-Einheiten aufgelöst und anschließend mit einem Gemisch von Thrombin und Calciumchlorid zur Gerrinnung gebracht. Diese Mischung gewährleistet einen festen Wundverschluß und auch weitgehend Schutz gegen zu schnelle Fibrinolyse.

Gegenstand der Erfindung ist weiterhin ein Verfahren zur Herstellung einer Fibrinogen- Zubereitung, dadurch gekennzeichnet, daß man eine Fibrinogenlösung bei einem pH-Wert von 5 bis 8 so lange auf einer Temperatur von 0 bis 15°C hält, bis die Fibrinogenpolymere ausgefallen sind, diese abtrennt, Arginin zusetzt und zur Trockene bringt.

Die erfindungsgemäße feste Fibrinogenzubereitung löst sich schneller auf und zu höheren Konzentrationen als die nach dem Stand der Technik hergestellten. Es können wässrige Lösungen bei Raumtemperatur erhalten werden, die bis zu 14 g/100 ml Fibrinogen enthalten. Löst man bei 37°C beispielsweise, dann können noch höhere Konzentrationen erreicht werden.

Da die Klebefestigkeit solcher Fibrinogenlösungen, wenn sie als Gewebeklebbar verwendet werden, mit dem Gehalt an Fibrinogen zunimmt, werden mit Gewebeklebern aus erfindungsgemäß hergestellten Konzentraten höhere Festigkeiten erhalten.

Dies kann im Rattenhautatreißtest gezeigt werden: Einem narkotisierten Versuchstier wird ein kreisförmiges Hautstück ausgestanzt. Dieses Hautstück wird mit einem Gemisch aus einem Volumenteil der erfindungsgemäßen Fibrinogenlösung und einem Volumenteil Thrombinlösung (400 NIH-Einheiten/ml) bestrichen und auf die Stanzwunde gepreßt, 15 Minuten dort belassen und anschließend das Abreißgewicht bestimmt.

Tabelle:
Abhängigkeit der Klebefestigkeit von der Fibrinogenkonzentration im Rattenhautreißtest

| | Abreißgewich in g; Mittelwert aus 10 Versuchen | |
| Fibrinogenkonzentration % | x | s |
| --- | --- | --- |
| 12 | 206 | 39 |
| 8 | 132 | 36 |
| 4 | 113 | 51 |
| 0 (Kontrolle) | 16 | 12 |

Ein weiterer Vorteil in der beschriebenen Weise hergestelter Fibrinogenkonzentrate ist ihre Stabilität nach Rekonstitution. Da in Kyropräzipitaten normalerweise Beimengungen von Prothrombinfaktoren und Plasminogen vorhanden sind, ist die Stabilität von Kryopräzipitatlösungen bei Raumtemperatur auf 2 bis 4 Stunden begrenzt. Fibrinogenkonzentrate, die nach dem folgenden Beispiel 2 hergestellt werden, sind dagegen über einen Arbeitstag bei Raumtemperatur stabil.

Die folgenden Beispiele sollen die Erfindung erläutern.

Beispiel 1
Herstellung eines Fibrinogenkonzentrates aus Kryopräzipitat

Ein Kryopräzipitat aus Citratplasma wurde in einer 0,01 M Na-Citratlösung von pH 8,5 enthaltend 1% (w:v) Arginin bei 37°C gelöst. Der nicht gelöste Anteil wurde in einer Ultrazentrifuge (Beckman J 21-B, Rotor JA 14) abzentrifugiert (45 min, 15.000 UpM) und verworfen.

Nach Einstellen der Fibrinogenkonzentration auf etwa 5% (w:v) Protein wurde der pH-Wert zwischen 7,0 und 9,0 gehalten. Nach Sterilfiltrieren, Abfüllen und Fluten des Behälters mit 100 Vol% Kohlendioxid erhielt man ein Lyophilisat, aus dem bei Raumtemperatur wässrige Fibrinogenlösungen mit bis zu 12% (w:v) Fibrinogen hergestellt werden konnten.

Beispiel 2
Herstellung eines Fibrinogenkonzentrates aus einem Fibrinogenniederschlag

6 kg eines aus einem Kryopräzipitat aus Citratplasma mit Hilfe einer 2 M Glycinlösung gefällten Fibrinogenniederschlages wurden mit 35 l Puffer (0,15 M NaCl, 0,01 M Citrat, 1 M Glycin) von pH 8.0 gewaschen und der Überstand abzentrifugiert. Der Rückstand wurde in 9 l 0,01 M Citrat und 0,15 M NaCl von pH 8,0 bei 37°C gelöst. Der pH-Wert muß unterhalb 8 liegen. Die Lösung wurde über Nicht bei 8 bis 10°C stehengelassen und der Niederschlag bei 8°C abzentrifugiert.

Der Überstand wurde zweimal gegen einen Puffer von pH 8, der aus 0,05 M NaCl, 0,005 M Citrat, 1 g/100 ml L-Arginin und 0,8 g/100 ml L-Leucin besteht, dialysiert.

Nach der Dialyse werden bei Einsatz als Fibrinkleber 60 E Faktor XIII/ml und 10 mg Human-Albumin pro ml zugesetzt.

Die Lösung wurde sterilfiltriert, lyophilisiert und das Produkt mit einer Mischung von 50 Vol% Kohlendioxid und 50 Vol% Stickstoff überlagert.

Die Lyophilisate können für intravenöse Anwendung in der Abfüllkonzentration von ca. 2—3% Fibrinogen gelöst werden; für den Einsatz als Fibrinkleber löst man in entsprechend weniger Lösungsmittel, so daß Konzentrationen zwischen 8—10% Fibrinogen erreicht werden.

**Patentansprüche für die Vertragsstaaten: BE CH DE FR GB IT LI LU NL SE**

1. Eine feste Fibrinogenzubereitung, dadurch gekennzeichnet, daß sie neben Fibrinogen Arginin enthält.

2. Eine Zubereitung nach Anspruch 1, dadurch gekennzeichnet, daß sie 0,05 bis 5 gewichtsprozent Arginin enthält.

3. Eine Zubereitung nach Anspruch 1, dadurch gekennzeichnet, daß sie 0,05 bis 5 Gewichtsprozent Arginin und mindestens 0,1 bis 5 Gewichtsprozent einer Aminosäure mit hydrophober Seitenkette oder einer wasserlöslichen Fettsäure enthält.

4. Eine Zubereitung nach einem der Ansprüche 1 bis 3, dadurch gekennzeichnet, daß sie zusätzlich Faktor XIII und/oder Aprotinin enthält.

5. Eine Zubereitung nach einem der Ansprüche 1 bis 4, dadurch gekennzeichnet, daß sie in einem

Behälter unter einer Gasatmosphäre mit mindestens 20 Volumenprozent Kohlendioxid vorliegt.

6. Verfahren zur Herstellung einer Zubereitung nach einem der Ansprüche 1 bis 5, dadurch gekennzeichnet, daß man eine Fibrinogen enthaltende Lösung bei einem pH-Wert zwischen 6 und 8 so lange auf einer Temperatur von 0—15°C hält, bis die Fibrinogenpolymeren ausgefallen sind, diese abtrennt, Arginin und gegebenenfalls eine Aminosäure mit hydrophober Seitenkette oder eine wasserlösliche Fettsäure und gegebenenfalls Faktor XIII und/oder Aprotinin zusetzt, trocknet und gegebenenfalls mit einem Kohlendioxid enthaltenden Gas überschichtet.

7. Verwendung einer Fibrinogenzubereitung nach einem der Ansprüche 1 bis 6 in einem Gewebekleber, der zusätzlich Thrombin und Calciumionen enthält.

8. Fibrinogenzubereitung nach einem der Ansprüche 1, 2, 3 und 5 als Fibrinogenkonzentrat für eine intravenöse Anwendung am Menschen.

**Patentansprüche für den Vertragsstaat: AT**

1. Verfahren zur Herstellung einer festen Fibrinogenzubereitung, dadurch gekennzeichnet, daß man eine Fibrinogen enthaltende Lösung bei einem pH-Wert zwischen 6 und 8 so lange auf einer Temperatur von 0—15°C hält, bis die Fibrinogenpolymeren ausgefallen sind, diese abtrennt, Arginin und gegebenenfalls eine Aminosäure mit hydrophober Seitenkette oder eine wasserlösliche Fettsäure und gegebenenfalls Faktor XIII und gegebenenfalls Aprotinin zusetzt, trocknet und gegebenenfalls mit einem Kohlendioxid enthaltenden Gas überschichtet.

2. Verwendung einer Fibrinogenzubereitung nach Anspruch 1 zur Herstellung eines Gewebeklebers, der zusätzlich Thrombin und Calciumionen enthält.

3. Fibrinogenzubereitung nach Anspruch 1 als Fibrinogenkonzentrat für eine intravenöse Anwendung am Menschen, wobei der Zubereitung kein Faktor XIII und kein Aprotinin zugesetzt wird.

**Revendications pour les Etats contractants: BE CH DE FR GB IT LI LU NL SE**

1. Préparation de fibrinogène solide, caractérisée en ce qu'elle contient, en plus du fibrinogène, d'arginine.

2. Préparation suivant la revendication 1, caractérisée en ce qu'elle contient 0,05 à 5% en poids d'arginine.

3. Préparation suivant la revendication 1, caractérisée en ce qu'elle contient 0,05 à 5% en poids d'arginine et au moins 0,1 à 5% en poids d'un amino-acide ayant une chaîne latérale hydrophobe ou d'un acide gras soluble dans l'eau.

4. Préparation suivant l'une des revendications 1 à 3, caractérisée en ce qu'elle contient en outre du facteur XIII et/ou de l'aprotinine.

5. Préparation suivant l'une des revendications 1 à 4, caractérisée en ce qu'elle se trouve dans un récipient sous une atmosphère gazeuse contenant au moins 20% en volume d'anhydride carbonique.

6. Procédé de préparation d'une préparation selon l'une des revendications 1 à 5, caractérisé en ce qu'on maintient à une température de 0—15°C une solution contenant du fibrinogène à un pH entre 6 et 8 jusqu'à ce que les polymères du fibrinogène aient précipité, en ce qu'on les sépare, en ce qu'on ajoute d'arginine et, le cas échéant, un aminoacide avec une chaîne latérale hydrophobe ou un acide gras soluble dans l'eau et, le cas échéant, du facteur XIII et/ou de l'aprotinine, en ce qu'on sèche et recouvre le cas échéant d'un gaz contenant de l'anhydride carbonique.

7. Utilisation d'une préparation de fibrinogène suivant l'une des revendications 1 à 6 dans une colle pour tissus qui contient en outre de la thrombine et des ions calcium.

8. Préparation de fibrinogène suivant l'une des revendications 1, 2, 3 et 5 comme concentré de fibrinogène pour une utilisation par voie intraveineuse chez l'homme.

**Revendications pour l'Etat contractant: AT**

1. Procédé de préparation d'une préparation de fibrinogène solide, caractérisée en ce qu'on maintient une solution contenant du fibrinogène à un pH entre 6 et 8 à une température de 0 à 15°C jusqu'à ce que les polymères du fibrinogène aient précipité, en ce qu'on les sépare, en ce qu'on ajoute d'arginine et, le cas échéant, un aminoacide ayant une chaîne latérale hydrophobe ou un acide gras soluble dans l'eau et le cas échéant du facteur XIII, et le cas échéant de l'aprotinine, en ce qu'on sèche et recouvre le cas échéant d'un gaz contenant de l'anhydride carbonique.

2. Utilisation d'une préparation de fibrinogène suivant la revendication 1 pour la préparation d'une colle à tissus, qui contient en outre de la thrombine et des ions calcium.

3. Préparation de fibrinogène suivant la revendication 1 en tant que concentré de fibrinogène pour une utilisation intraveineuse chez l'homme, à laquelle on n'a ajouté ni facteur XIII ni aprotinine.

**Claims for the Contracting States: BE CH DE FR GB IT LI LU NL SE**

1. A solid fibrinogen formulation which contains, in addition to fibrinogen, arginine.

2. A formulation as claimed in claim 1, which contains 0.05 to 5 per cent by weight of arginine.

3. A formulation as claimed in claim 1, which contains 0.05 to 5 percent by weight of arginine and at least 0.1 to 5 per cent by weight of an aminoacid having a hydrophobic side chain or of a water-soluble fatty acid.

4. A formulation as claimed in one of claims 1 to 3 which additionally contains factor XIII and/or aprotinin.

5. A formulation as claimed in one of claims 1 to 4, which is present in a container under a gas atmosphere containing at least 20 per cent by volume of carbon dioxide.

6. A process for the preparation of a formulation as claimed in one of claims 1 to 5, which comprises maintaining a solution containing fibrinogen at a pH between 6 and 8 and at a temperature of 0—15°C until the fibrinogen polymers have precipitated out, separating these off, adding arginine and optionally an aminoacid having a hydrophobic side chain or a water-soluble fatty acid and optionally factor XIII and/or aprotinin, drying and optionally covering with a gas containing carbon dioxide.

7. The use of a fibrinogen formulation as claimed in one of claims 1 to 6 as a tissue adhesive which additionally contains thrombin and calcium ions.

8. Fibrinogen formulation as claimed in one of claims 1, 2, 3 and 5 as fibrinogen concentrate for an intravenous administration to humans.

**Claims for the Contracting State: AT**

1. A process for the preparation of a formulation as claimed in one of claims 1 to 5, which comprises maintaining a solution containing fibrinogen at a pH between 6 and 8 and at a temperature of 0—15°C until the fibrinogen polymers have precipitated out, separating these off, adding arginine and optionally an aminoacid having a hydrophobic side chain or a water-soluble fatty acid and optionally factor XIII and/or aprotinin, drying and optionally covering with a gas containing carbon dioxide.

2. The use of a fibrinogen formulation as claimed in claim 1 as a tissue adhesive which additionally contains thrombin and calcium ions.

3. Fibrinogen formulation as claimed in claim 1 as fibrinogen concentrate for an intravenous administration to humans, whereby there is no factor XIII and aprotinin added to the formulation.